# EUROPEAN PATENT SPECIFICATION

(11) **EP 2 734 151 B1**
(45) Date of publication and mention of the grant of the patent: **31.01.2018**
(21) Application number: 12740332.7
(22) Date of filing: 17.07.2012
(51) Int. Cl.: A61F 2/16

(54) **CASSETTE FOR AN INTRAOCULAR LENS AND INJECTOR DEVICE FOR AN INTRAOCULAR LENS**
KASSETTE FÜR EINE INTRAOKULARLINSE UND INJEKTOR FÜR EINE INTRAOKULARLINSE
CASSETTE DESTINÉE À UNE LENTILLE INTRAOCULAIRE ET DISPOSITIF D'INJECTEUR POUR UNE LENTILLE INTRAOCULAIRE

(30) Priority: 19.07.2011 GB 201112580
(43) Date of publication of application: 28.05.2014
(73) Proprietor: Carl Zeiss Meditec SAS, 17053 La Rochelle Cedex 9 (FR)
(72) Inventor: RAQUIN, Vincent, 17000 La Rochelle (FR); PANKIN, Dmitry, 13355 Berlin (DE); RATHERT, Brian, 8500 Frauenfeld (CH)
(74) Representative: Hofstetter, Schurack & Partner
(86) International application number: PCT/EP2012/064001
(87) International publication number: WO 2013/011024

(56) References cited:
- WO-A2-2004/060099
- DE-A1-102005 004 598
- US-A1- 2008 147 080

## Description

### Technical field

The invention relates to a cassette for an intraocular lens, which has a receiving container for the intraocular lens. This receiving container has a front and a rear face viewed in axial direction. Moreover, the invention also relates to an injector device for an intraocular lens with such a cassette.

### Prior art

Cassettes for intraocular lenses are known in manifold configuration. Thus, for example from WO 2010/031196 A1, such a cassette is known, which has a lid part and a bottom part. An intraocular lens is insertable into this cassette. The cassette has a front and a rear opening such that a channel is formed. A piston can be introduced into an injector device through the rear opening, which then pushes the intraocular lens out of the cassette through the front opening.

In known implementations of cassettes it is usual that they are for example manufactured and provided of plastic. Usually, a container is provided separately thereto, in which the intraocular lens is sterilely disposed. Only when a surgical procedure is imminent, this container with the intraocular lens, which is filled with liquid, is opened, the intraocular lens is removed from it and it is only then introduced into the cassette. The cassette is then attached to the injector device and the already above mentioned piston pushes the lens out of the cassette.

Thus, first, the lens is provided and supplied in a separate transport container. The cassette is supplied separately thereto. First, then, an elaborate introduction process of the lens into the cassette has to be effected. Therein, damage to the lens can also occur and optionally the sterility can also be affected.

Moreover, the user is also required to first go through a certain learning phase in order to be able to perform the correct introduction of the lens into the cassette on the one hand and the correct mounting of the cassette to the injector device on the other hand without error.

From document DE 10 2005 004 598 A1 an apparatus to implant an intraocular lens is known.

Moreover, document WO 2004/060099 A2 discloses a packaging for storing a contact lens in sterile fluid.

Furthermore, document US 2008/0147080 A1 shows a cassette for an intraocular lens in which openings are closed by foils.

### Presentation of the invention

It is the object of the present invention to provide a cassette for an intraocular lens as well as an injector device for an intraocular lens, by which the handling of an intraocular lens can be effected simply and with less effort.

This object is solved by a cassette and an injector device having the features of the independent claims.

A cassette according to the invention is formed for receiving an intraocular lens. The cassette includes a receiving container for the intraocular lens. The receiving container has a front and a rear face viewed in axial direction of the receiving container. The front face has an opening, which is closed by a foil attached to the receiving container as a cover, and/or the rear face has an opening, which is closed by a foil attached to the receiving container as a cover. Thus, the cassette is formed with its two openings in order that upon arrangement on the injector device a piston of the injector device can penetrate the cassette through one of the openings and push the intraocular lens then located therein out of the cassette. The cassette thus does not constitute a transport container for an intraocular lens, but a module, which can directly be attached to the injector device for further treatment of the intraocular lens located therein or is integrally formed in the injector device itself.

In that at least one of the openings on the face of the receiving container is closed by a very thin foil, it can be allowed that the intraocular lens is already directly inserted in the cassette and the cassette is therefore multi-functionally formed. This, because it therefore also has the integral function of the transport container. Thus, the cassette according to the invention is a component constituting the transport container itself on the one hand, constituting also exactly that component on the other hand, which is or can be connected to the injector device and which then allows in the state connected to the injector device that the intraocular lens located therein can be pushed out of the cassette. By the foil, a mechanically stable closure is allowed on the one hand, which also allows the sterile storage of the intraocular lens in the cassette on the other hand.

Preferably, a balanced saline solution or pure water is disposed in the interior of the receiving container. The intraocular lens located therein is thereby sterilely stored.

By the attachment of a foil to at least one of the faces, this foil can also be processed simply and with low effort, in order to then be able to bring out the intraocular lens from the receiving container in the following. This, in particular when this intraocular lens is to be pushed out of this cassette by a piston of the injector device.

Thus, the cassette according to the invention integrates a plurality of functions in a single component, which requires a plurality of separate components in conventional configurations and requires additional handling effort on the other hand.

Thus, in particular after completion of the cassette, the intraocular lens is introduced into the receiving container and the balanced saline solution is introduced. Thereafter, at least one of the openings is closed with the foil. The thus provided cassette therefore includes a hermetically sealed receiving container, in which the intraocular lens is already sterilely stored and integrated. A so formed cassette can then furthermore already be integrated in an injector device such that already the complete injector device can be provided and then subsequently delivered and optionally supplied to a surgeon. In such a configuration, thus, the amount of work and in particular the mounting effort is minimized.

It is particularly advantageous if a foil is a coextruded foil. For such a specific foil it is possible in a manner particularly to be emphasized that a hermetically tight seal of the opening with the foil can be ensured on the one hand. On the other hand, it is thereby achieved that a particularly sterile barrier is also generated by the foil such that undesired contaminants cannot enter the interior of the receiving container. Moreover, this coextruded foil further ensures the desired flexibility with regard to the subsequent removal or piercing when the intraocular lens is to be pushed out of the cassette.

It is advantageous that the coextruded foil has an aluminum layer. Herein, aluminum is a material particularly to be emphasized in order to be able to ensure the sterile barrier of the foil.

Advantageously, it is provided that the coextruded foil has propylene, in particular includes a polypropylene layer. In particular, this material component is suitable for hermetically sealing and attaching to the receiving container. Therein, the polypropylene material can be particularly sealingly connected to the material in the receiving container.

Preferably, the coextruded foil is formed to the effect that it has a polypropylene layer, on which an aluminum layer is formed. The aluminum layer is then preferably further covered with a varnish or other coating.

Preferably, it is provided that the foil has a thickness between 0.30 mm and 6 mm, preferably between 0.3 mm and 2 mm, preferably between 0.3 mm and 1 mm, preferably between 0.45 mm und 0.55 mm, in particular 0.5 mm. Such a thin foil is highly flexible on the one hand and can be easily and safely torn off with a suitable handling tool without it tearing apart. Similarly, it can be specifically and desirably pierced with a suitable handling tool without it undesirably being disrupted.

On the other hand, it also ensures a sufficient thickness to be able to prevent undesired piercing or detaching. In particular, a sufficient thickness is provided in order to be able to attach the foil mechanically safely and sealingly to the receiving container and to be able to ensure the sterility for the interior of the receiving container.

The foil is connected to a tear-off element, which is movable relatively to the receiving container for tearing off the foil from the receiving container. Thereby, a particularly simple handling means is ensured, which thus is similarly already formed and present integrally with the cassette. Here too, thus, assembly effort is omitted. On the other hand, the tear-off element is disposed and connected to the foil such that an intuitively correct movement sequence is performed and thus detaching and tearing-off of the foil is automatically effected in the preferred direction. The above mentioned advantages are thereby once again supported.

Preferably, the tear-off element is designed as a plate-like grip part. Thereby, a user can safely grip and hold the tear-off element. Thereby, slip-off is prevented.

Preferably, it is provided that a foil is formed in two layers on an opening. This is in particular designed to the effect that the layer closer to the receiving container is fixedly connected thereto such that the seal and sterile barrier is ensured. The second layer facing away from the receiving container is in particular designed to the effect that it is in particular not fixedly connected to the first layer in the superposition region with the first layer. Thus, a certain relative mobility to the first layer is ensured.

Preferably, the second layer is connected to the tear-off element. Thus, especially with regard to the attachment of the foil to the tear-off element on the one hand and the receiving container, a safe function chain with regard to the removal of the foil from the receiving container can be ensured. The tear-off operation is thus allowed in particularly simple and target-aimed manner, such that tearing-off of the foil from the receiving container is ensured without tearing the foil apart.

The foil is integrally formed, in particular formed as a strip. A bend is formed at the end of the foil facing away from the tear-off element and a second foil layer outside with respect to the opening of the receiving container is connected to the tear-off element with the free end facing away from the bend. The above mentioned advantages are therein achieved in a manner particularly to be emphasized. Because, if the tear-off element is pulled away from the cassette, thus, the location of the bend is virtually pulled further and further and a particularly suitable force introduction to the first foil layer with regard to its detachment from the receiving container can be ensured. A particularly uniform and unidirectional detachment of the first foil layer from two opposing attachment lines on the receiving container is thereby ensured.

Preferably, a foil is thermally welded to the receiving container. Thereby, a particularly simple yet very sealing connection can be provided. Especially if the receiving container is a plastic material and the polypropylene material of the coextruded foils is heated, a particularly sealing connection between the plastic materials can be achieved.

Preferably, it is provided that a displaceable elastic damper element (or soft cushion) is disposed in the receiving container on the opening of the receiving container constituting an input-side opening. Therein, the input-side opening constitutes that opening of the receiving container, which faces a piston of an injector device for pushing-out the intraocular lens, if the cassette is disposed in the injector device. Preferably, this damper element is positioned immediately on the inner side of the foil, which closes this input-side opening. This damper element is thus disposed between this foil, which closes the input-side opening and the intraocular lens located in the receiving container.

The damper element is preferably formed of an elastomer, in particular silicone. By such an integrated damper element, a particular advantageousness can be achieved in a specific configuration of the cassette and thus also of an injector device such that exactly then a very hard and stiff piston can be used, by which the foil closing the input-side opening of the cassette can be pierced very specifically in desired manner. In that the damper element is further located behind, thus, a soft component is provided, which then serves as an adapter in order to contact the intraocular lens and push it out of the cassette. Thus, it can be avoided that the very hard and stiff piston directly contacts the intraocular lens and optionally would be able to damage it.

Thus, with this damper element, a buffer for the piston optionally harder striking the receiving container is virtually formed on the one hand. On the other hand, the damper element then ensures a particularly gentle contact with the intraocular lens in the further process of pushing the intraocular lens out of the cassette. On the other hand, a particularly suitable push-out piston provided with a hard front end can be used, as already mentioned, which in turn ensures the advantageousness of the safe and desired piercing of the foil of the cassette.

However, it is to be mentioned that such a damper element can also be disposed in the receiving container in any other implementation of the cassette. Thus, such a damper element can also be disposed on a cassette, in which one or both foils closing the openings on the face are removed with a tear-off element.

In the configuration of the cassette, different implementations can be provided. Here, it can be provided that for example both openings are closed with each one foil. In such a configuration, it can then be provided that both foils are connected to a single tear-off element and with the movement of the tear-off element both foils are torn off the receiving container at the same time.

In another implementation, it can also be provided that each foil is connected to an own separate tear-off element.

Similarly, it can be provided that only one of the two foils is connected to a tear-off element and the connected foil is then removed from the receiving container by actuating the tear-off element. The second foil is then formed without such a tear-off element. It can then be designed to the effect that it can be pierced by a piston of the injector device. In particular, it can have a perforation structure for this such that piercing the foil is effected in very specific manner and at a very specific location.

In particular it is provided that thus the input-side opening is then formed without such a tear-off element. The output-side opening is then formed with this tear-off element.

In a further implementation it can be provided that the output-side opening of the cassette is formed without such a tear-off element. In particular, this foil then has a characterizing perforation structure. In such a configuration, it can then be provided that an injector tip of the injector device, which is formed as a separate component, has a perforation element. Upon attachment of the injector tip to the injector tube, then, the foil attached to the output side is automatically pierced at the desired location and in the desired manner by this perforation element. In such a configuration, it can be provided that the foil formed on the input-side opening is connected to a tear-off element. However, preferably, it can here be provided that the foil attached to the input-side opening is also formed without such a tear-off element and also in particular has a specific perforation structure. It can then be pierced by the mentioned piston of the injector device.

Preferably, the cassette includes a container with viscoelastic material, which is subsequently disposed on the receiving container, in particular the container is closed in covering manner in axial direction on both sides with a foil, in particular a coextruded foil.

Preferably, at least one foil has a defined perforation structure already mentioned above. This perforation structure can in particular be crossed lines, especially two crossed lines, especially a cross with lines are perpendicular to each other.

Such a cross structure, which is in particular then also centrally formed on a foil, ensures a particularly safe and centered piercing of a foil, thereby also providing a maximum tear-open geometry to be able to output the lens simply and via a perforation opening as large as possible.

Especially the cassette is a receiving container to store a hydrophilic lens in water medium (pure or salted water).

Furthermore, the invention relates to an injector device for an intraocular lens with a cassette according to the invention or an advantageous configuration thereof. In particular, the injector device includes an injector tube, on which the cassette is disposed.

In particularly advantageous manner, it is provided that the injector device already has the cassette in integrated manner. Thus, a highest degree of integration of components is virtually achieved, which ensure the transport of an intraocular lens to a surgeon on the one hand and the handling by a surgeon on the other hand. Thereby, assembly effort for the cassette to the injector device on the one hand, in particular for the medical personnel, and any effort for introducing the intraocular lens from a separate transport container into the cassette, in particular by the medical personnel, is no longer required.

Preferably, the injector device has an injector tip formed with a perforation edge or a perforation element. With this perforation edge, the foil of the cassette facing the injector tip is automatically severable upon arrangement of the injector tip on an injector tube of the injector device. The above already mentioned advantages correspondingly apply here.

Preferably, the injector device has a piston for pushing the intraocular lens out of the cassette, which also has a perforation means or a perforation element, by which upon the piston striking a foil of the cassette facing the piston, this foil is automatically severable. Here too, the already above mentioned advantages apply in analogous manner.

Preferably, it is provided that the cassette is steam sterilized and the injector tip and the injector tube are sterilized with ethylene oxide.

Preferably, it is provided that the injector device is provided and deliverable with a cassette integrated therewith in a closed transport container. As already above mentioned, this is particularly advantageous. Because the medical personnel then only has to open this transport container and can remove the overall injector device thereupon and begin with the further handling of the injector device without further assembly process or removal process of the intraocular lens from a separate transport container or introduction into the cassette.

Further features of the invention are apparent from the claims, the figures and the description of figures. The features and feature combinations mentioned above in the description as well as the features and feature combinations shown in the description of figures alone and/or the features and feature combinations shown only in the figures alone are usable not only in the respectively specified combination, but also in other combinations or alone, without departing from the scope of the invention.

### Brief description of the drawings

Embodiments of the invention are explained in more detail below by way of schematic drawings.

There show:
- Fig. 1: a perspective illustration of an embodiment of a cassette according to the invention;
- Fig. 2: a perspective illustration of an embodiment of an injector device according to the invention;
- Fig. 3: a perspective illustration of a partial section of the injector device according to Fig. 1 in a first handling state;
- Fig. 4: a perspective illustration of a partial section of the injector device according to Fig. 2 in a second handling state;
- Fig. 5: a perspective illustration with a cross-section of a partial section of the injector device according to Fig. 2 in a third handling state;
- Fig. 6: a perspective partial section with a sectional view of the injector device according to Fig. 3 in a fourth handling state;
- Fig. 7: a perspective illustration of partial components of an injector device according to an embodiment not comprised of the invention;
- Fig. 8: a perspective illustration with a sectional view of the implementation according to Fig. 7;
- Fig. 9: a perspective illustration with a sectional view of a further embodiment of an injector device not comprised of the invention;
- Fig. 10: a perspective illustration of the implementation in Fig. 9;
- Fig. 11: a perspective illustration of a partial section of a further embodiment of an injector device according to the invention;

### Preferred implementation of the invention

In the embodiment, similar or functionally equivalent elements are provided with the same reference characters.

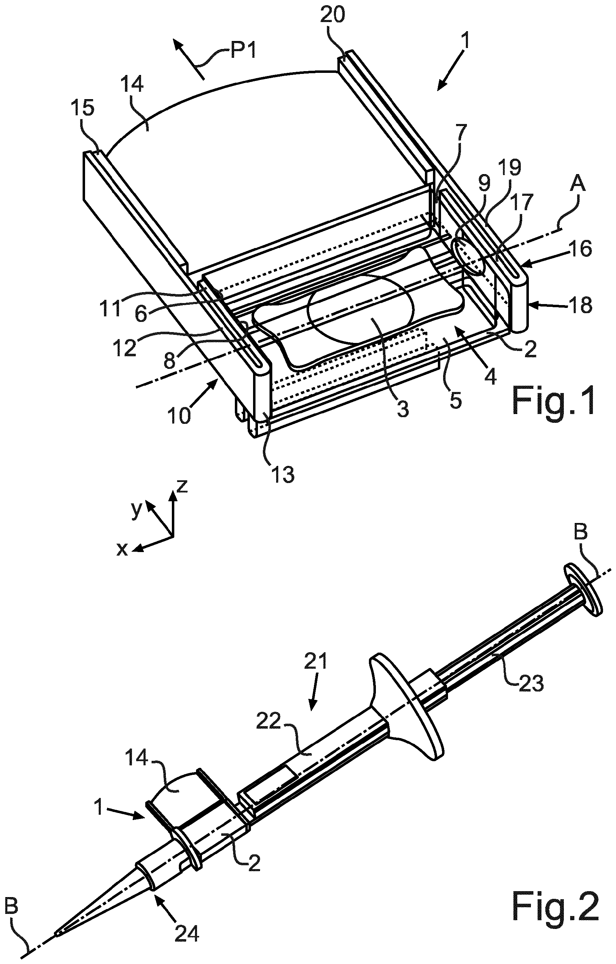

In Fig. 1, in a perspective illustration, a first embodiment of a cassette 1 is shown. The cassette 1 includes a receiving container 2 rectangular in the embodiment, in which an intraocular lens 3 is disposed. Thereto, the receiving container 2 includes an internal space 4, which is filled with a balanced saline solution 5, or water medium.

The receiving container 2 includes a front face 6 and a rear face 7 viewed in direction of its longitudinal axis A. An opening 8 is formed in the front face 6. Similarly, an opening 9 is formed in the rear face 7. The openings 8 and 9 are dimensioned such that the intraocular lens 3 can be correspondingly input and output.

Thus, the cassette 1 is formed for receiving the intraocular lens 3. Moreover, it is also provided for directly attaching to an injector for introducing the intraocular lens 3 into an eye.

Preferably, it is provided that this cassette 1 is integrally disposed on such an injector. Therein, the openings 8 and 9 disposed in axial direction are designed such that a piston of the injector device can be pushed in via the opening 8, and thereby the intraocular lens 3 can be pushed out of the receiving container 2 via the opening 8.

Moreover, the cassette 1 includes a first coextruded foil 10. This coextruded foil 10 includes polypropylene as a first layer and an aluminum layer attached thereon. It is then in turn covered by a varnish layer or the like. The coextruded foil 10 extends across the entire width (y direction) of the receiving container 2 with a first layer 11. With regard to its height (z direction), the foil 10 is formed substantially corresponding to the height of the receiving container 2. This first layer 11 of the foil 10 is directly attached to the receiving container 2, in particular sealingly disposed thereon by thermally welding. Thus, the front opening 8 is sterilely sealed by this foil 10 in particular with the first layer 11.

Therein, a sufficiently tight attachment to the plastic of the receiving container 2 is ensured by the polypropylene layer. By the aluminum layer it is ensured that the intraocular lens 3 and the interior of the receiving container 2 can be maintained sterile.

The foil 10 strip-like in the embodiment moreover includes a second layer 12. The foil 10 is integrally formed and the two layers 11 and 12 are produced in that the strip-like foil 10 is bent at a bend 13 and thereby the two-layeredness on the front face 6 is produced. This second layer 12 extends in the y direction beyond the length of the first layer 11 and is attached to a plate-shaped tear-off element 14. With regard to its length (x direction), the plate-shaped tear-off element 14 is formed with a length corresponding to the length of the receiving container 2. As is apparent, the tear-off element 14 is disposed abutting a longitudinal side of the receiving container 2.

The second layer 12 is attached to a face of a plate-like receiving part 15 of the tear-off element 14. This layer 12 also extends over the entire length of this receiving part 15.

On the opposing side, an analogous configuration is formed. There too, a foil 16 being a coextruded foil of polypropylene and aluminum is disposed such that the opening 9 is sealingly and sterilely closed. Here too, a first layer 17 is directly disposed on the receiving container 2 and a second layer 19 of the integral foil 16 is formed via a bend 18. Here too, this second layer 19 is then attached to a receiving element 20 of the tear-off element 14 on the outside.

The foils 10 and 16 have a thickness between 0.45 mm and 0.55 mm, in particular 0.5 mm, with respect to a layer.

For removing the foils 10 and 16, the tear-off element 14 is gripped and peeled off in the direction of the arrow P1 and thus in particular in the x-y plane. Thereby, the bend 13 or 18 is pulled further upwards and thus the first layers 11 and 17 attached to the receiving container 2 are detached from the receiving container 2.

In particular it is provided that the thus shown cassette 1 is already provided as a prefabricated unit and corresponding module and can be correspondingly supplied and transported. Then, it can be provided that it is plugged or locked to a present injector device or attached thereto in correspondingly other manner in situ.

In thus particularly advantageous manner, it is no longer required that the intraocular lens 3 is provided and delivered in a separate transport container and a cassette is provided and delivered as a separate component and then the intraocular lens has to be introduced into the cassette only in situ in elaborate and error-prone manner as well as optionally unhygienic manner. Exactly this all can therefore be prevented if an implementation according to Fig. 1 is formed.

In Fig. 2, in a perspective illustration, an embodiment of an injector device 21 according to the invention is shown. This injector device 21 is constructed syringe-like and includes an injector tube 22. A piston 23 is guided in it, which can be displaced in the direction of the longitudinal axis B. Moreover, the injector tube 22 is formed for receiving a cassette 1 as it has for example been shown and explained in Fig. 1. The front-side end of the injector device 21 constitutes an injector tip 24.

In the shown embodiment, it is in particular provided that the cassette 1 is integrated in the injector tube 22. Thus, it can be provided that the entire injector device 21 is already completely finished and delivered such that it is also no longer required with medical personnel in situ that the cassette 1 still has to be mounted to the injector tube 22.

In particularly advantageous manner, the injector device 21 is sterilized with ethylene oxide and the cassette 1 is steam sterilized.

As an alternative the complete device (injector and cassette) is gamma sterilized in one step, after complete packaging.

In preferred manner, the entire injector device 21 with the already disposed cassette 1 is stored in a transport container and then already finished delivered in situ for example to a surgeon with this transport container. The user then only has to open the transport container 1 and remove the injector device 21 shown in Fig. 2 from it.

By actuating the piston 23 and thus by pushing the piston 23 in the direction of the axis B, the piston tip not shown in Fig. 2 enters the receiving container 2. Then, the intraocular lens 3 located therein is pushed out of the receiving container 2 and thus also from the front opening 8 along the axis B of the injector device 21 and pushed into the injector tip 24. There, it then exits at the front cannula end in rolled or folded manner and can be introduced into the eye.

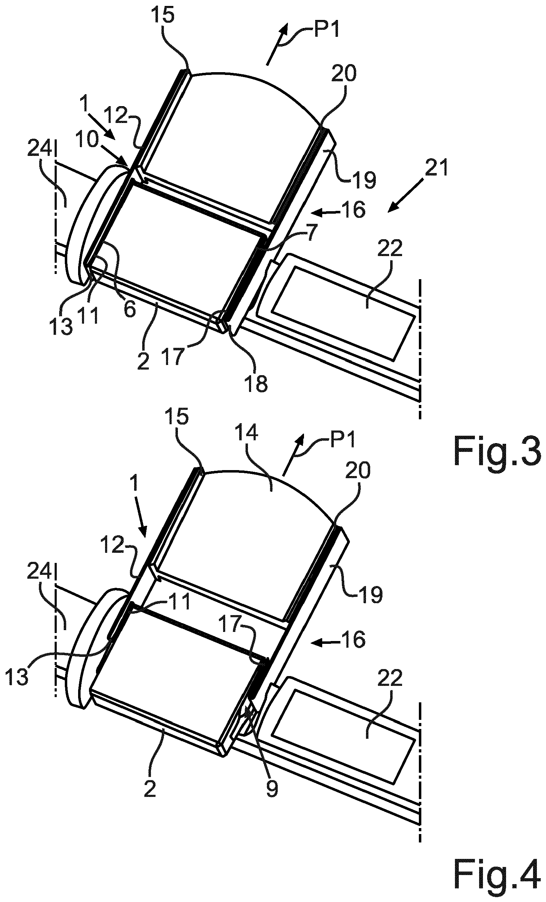

In Fig. 3, in perspective illustration, an enlarged section of the injector device 21 in the region of the cassette 1 is shown. Here, a state is shown, in which the cassette is minimally opened and thus the tear-off element 14 is only slightly pulled in the direction of the arrow P1. The openings 8 and 9 are still closed here.

In Fig. 4, a further perspective illustration of the partial section according to Fig. 3 is shown, wherein here the tear-off element 14 has already been further pulled in the direction of the arrow P1. Therein, it can be recognized that the first layers 11 and 17 of the foils 10 and 16 already have been separated halfway from the faces 6 and 7 of the receiving container 2. In this connection, the opening 9 is already perceivable.

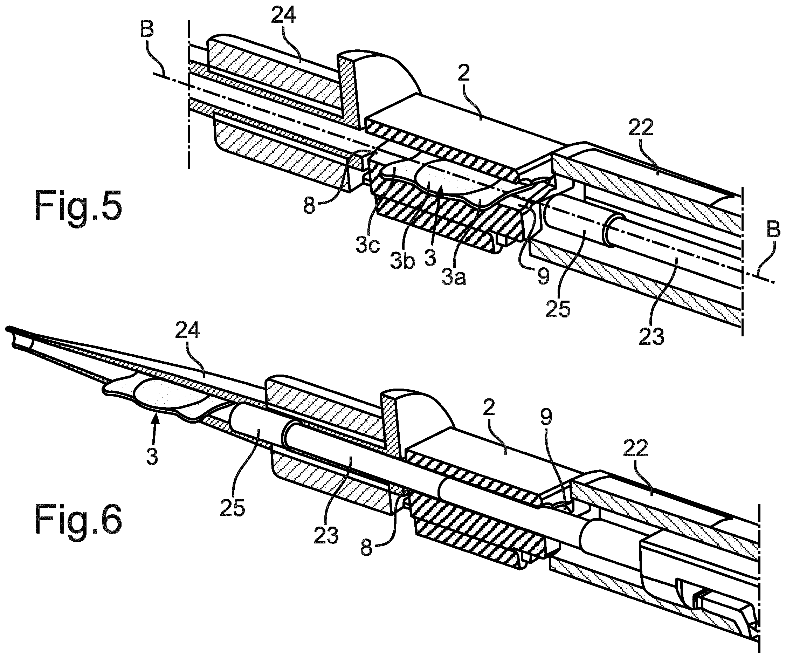

In Fig. 5, then, in a perspective illustration, which also shows a cross-section, the cassette 1 is shown in a state, in which the tear-off element 14 with the foils 10 and 16 is completely separated from the receiving container 2.

Moreover, it can be recognized that the front end of the piston 23 has a damping element 25 in particular formed of silicone. It then contacts the intraocular lens 3 on the way along the axis B, if the piston 23 has penetrated the receiving container 2 via the opening 9. Therein, a haptic part 3a of the lens 3 is in particular contacted. On the opposing side of an optic part 3b, then, a further haptic part 3c is similarly formed.

As already mentioned, the lens 3 is then pushed out of the receiving container 2 via the front opening 8 and introduced into the channel of the injector tip 24. This is shown in the illustration according to Fig. 6.

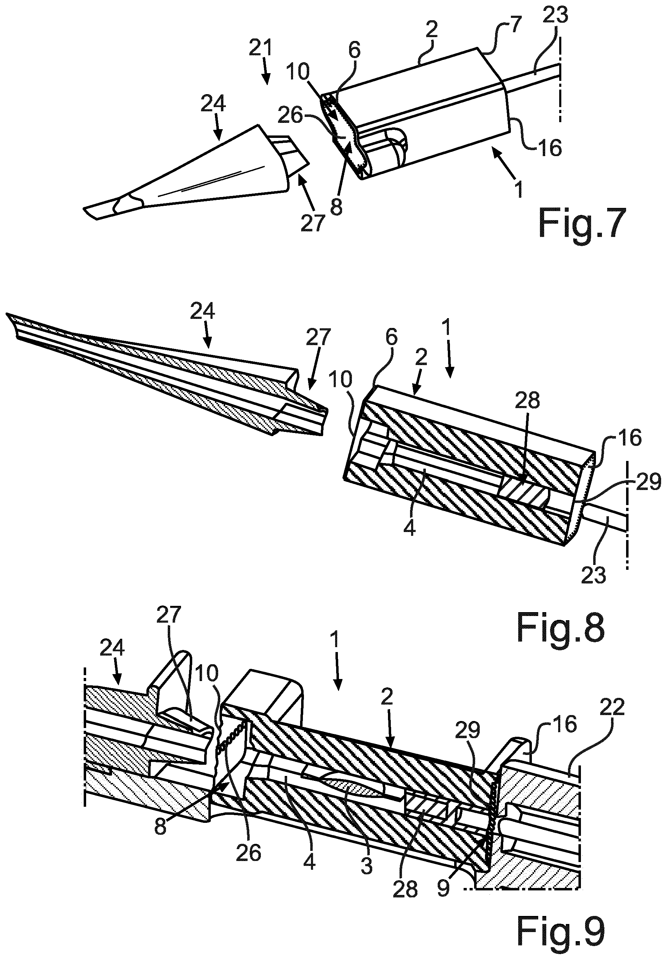

In Fig. 7, in a perspective illustration, a further embodiment not comprised of the invention of partial components of an injector device 24 is shown. In this configuration, a cassette 1 is provided, which has a receiving container 2. This cassette 1 does not include a tear-off element 14, as it is provided in Fig. 1. Rather, here it is provided that a coextruded foil 10 is again disposed on the face 6 on the receiving container 2 and an opening 8 is sterilely and sealingly closed. In this embodiment, it is provided that the foil 10 is only attached in a single layer and has a defined perforation structure 26. In this configuration an injector tip 24 of the injector device 21 includes a perforation element 27 on the side facing the cassette 1. It can have a very specifically defined perforation edge. Upon assembling the injector tip 24 to the cassette 1, thus, the perforation structure 26 is automatically severed by the perforation element 27. The access to the interior of the receiving container 2 and thus to the intraocular lens 3 is thereby given. The intraocular lens 3 can then be pushed out of the receiving container 2 via the opened foil 10 in the region of the pierced perforation structure 26.

Correspondingly, it can be provided that a corresponding foil 16 is also attached in a single layer on the opposing face 7 of the receiving container. This coextruded foil 16 too has a perforation structure. Then, it can be pierced with the front end of the piston 23.

In Fig. 8, a perspective illustration of the implementation in Fig. 7 is shown, wherein moreover a cross-section is also shown. Therein, the shape and geometry of the perforation element 27 can be recognized. For the sake of clarity, in Fig. 8, the intraocular lens 3 is not drawn. At this point, it is to be mentioned that a damper element 28 is disposed in the interior 4 of the receiving container 2, which is for example formed of silicone. It is disposed adjacent to the foil 16 and disposed between this foil 16 and the not shown intraocular lens 3. By this elastic damper element 28, which has the analogous function to the damper element 25 in Fig. 5 and Fig. 6, damage to the intraocular lens 3 upon pushing out is prevented. Because in a configuration as it is shown in Fig. 7 and Fig. 8, it is advantageous that the front end of the piston 23 is relatively hard and optionally also formed with a perforation edge. This to the effect that a perforation structure 29 formed in the foil 16 can be pierced. However, since such a hard and inflexible piston 23 could then damage the intraocular lens 3, the damper element 28 is of particular advantageousness. By the integral arrangement of the damper element 28 in the receiving container 2, here too, a functional integration is ensured and the attachment of such a damper element 28 to the piston 23, if this damper element 28 would not be present integrated in the receiving container 2, can no longer be forgotten.

In Fig. 9, a further embodiment not comprised of the invention of a cassette 1 on the one hand and of an injector device 21 on the other hand is shown.

In this configuration, a cassette 1 is also again formed without abrasion element for removing the foils 10 and 16. The shape of the receiving container 2 is here different from the previous embodiments. Thereby, different injector tips 24 and/or different injector tubes 22 can optionally be combined and connected to the cassette 1.

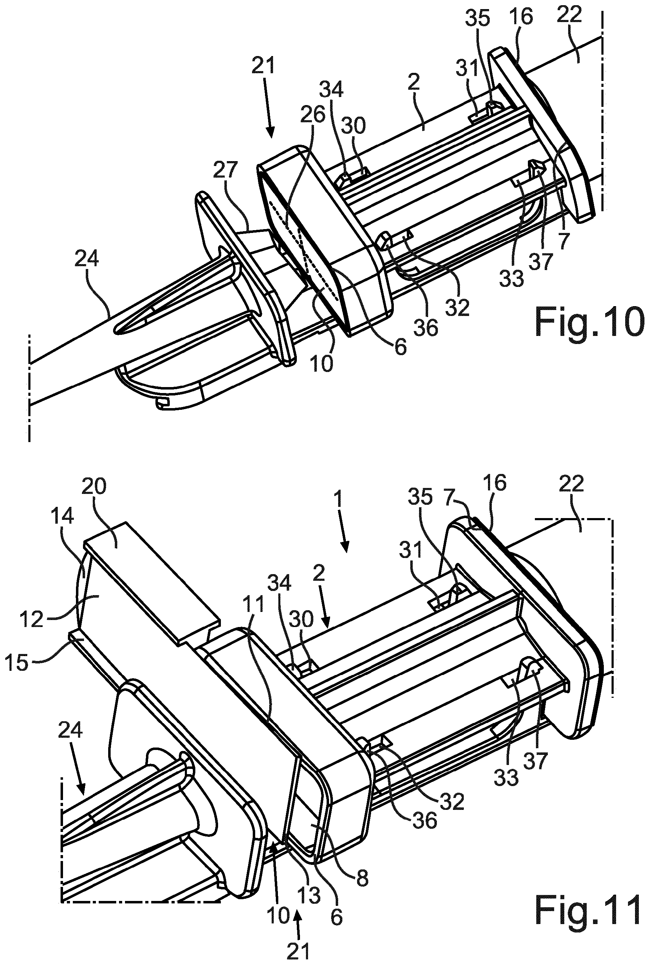

In Fig. 10, a perspective illustration of the embodiment according to Fig. 9 is shown. The perforation structure 26 as it is formed in the foil 10, is realized as a cross. The perforation structure 29 in the foil 16 is also correspondingly realized. As it can be appreciated, passages 30, 31, 32 and 33 are formed on the body of the receiving container 2. They are formed for guiding through and snapping by locking elements 34, 35, 36 and 37. Thereby, the cassette 1 can be disposed locking to the injector tube 22 in non-destructively detachable manner. Thereby, integral attachment is possible and the entire injector device 21 can be delivered already completely assembled. However, in particular, the injector tip 24 is not yet attached to the cassette 1 in its final position such that the perforation structure 26 then is not yet pierced.

In Fig. 11, in a perspective illustration, a further embodiment of a cassette 1 and a further embodiment of an injector device 21 are shown. In this configuration, unlike the implementations in Fig. 1 and Fig. 7 to Fig. 10, it is provided that a front opening 8 of the receiving container 2 facing the injector tip 24 is closed by a two-layer attached foil 10. In this configuration, the tear-off element 14 is not oriented in the x-y plane, but in the y-z plane. Therein, the second layer 12 of the foil 10 is attached to the center plate element of the tear-off element 14. The remaining arrangement and removal of the foil 10 from the front face 6 is analogous to the explanation in Fig. 1.

In contrast, the opening 9 facing away from the injector tip 24 is sterilely and sealingly covered with a foil 16 analogously to the configurations in Fig. 7 to Fig. 10, which is formed without such a tear-off element. Rather, analogously to the explanations in Fig. 7 to Fig. 10, a perforation structure 29 is formed here, which can be pierced by the piston 23.

## Claims

1. Cassette (1) for an intraocular lens (3), which has a receiving container (2) for the intraocular lens (3), which has a front (6) and a rear face (7) in the axial direction (A) of the receiving container (2), wherein
the front face (6) has an opening (8), which is closed by a foil (10) attached to the receiving container (2) as a cover, and/or the rear face (7) has an opening (9), which is closed by a foil (16) attached to the receiving container (2) as a cover,
**characterized in that**
said foil (10, 16) is connected to a tear-off element (14), which is movable relatively to the receiving container (2) for tearing off the foil (10, 16) from the receiving container (2), and
the foil (10, 16) is integrally formed and has a bend (13, 18) at the end facing away from the tear-off element (14), and a second foil layer (12, 19) outside with respect to the opening (8, 9) of the receiving container (2) is connected to the tear-off element (14) at the free end facing away from the bend (13, 18).

2. Cassette (1) according to claim 1,
**characterized in that**
said foil is a coextruded foil (10, 16).

3. Cassette (1) according to claim 2,
**characterized in that**
said coextruded foil (10, 16) comprising aluminum.

4. Cassette (1) according to claim 2 or 3,
**characterized in that**
said coextruded foil (10, 16) comprising polypropylene.

5. Cassette (1) according to anyone of the preceding claims,
**characterized in that**
the foil (10, 16) has a thickness between 0.3 mm and 6 mm, in particular 0,3 mm and 1 mm, in particular 0.5 mm.

6. Cassette (1) according to anyone of the preceding claims,
**characterized in that**
said foil (10, 16) is formed in two layers on an opening (8, 9).

7. Cassette (1) according to anyone of the preceding claims,
**characterized in that**
said foil (10, 16) is thermally welded to the receiving container (2).

8. Cassette (1) according to anyone of the preceding claims,
**characterized in that**
a displaceable elastic damper element (28) is disposed in the receiving container (2) on the opening (9) constituting an input-side opening for a piston (23) on an injector device (21).

9. Cassette (1) according to anyone of the preceding claims,
**characterized in that**
said foil (10, 16) has a defined perforation structure (26, 29).

10. Cassette (1) according to claim 9,
**characterized in that**
the perforation structure (26, 29) includes at least two crossed lines.

11. Injector device (21) for an intraocular lens (3), including a cassette (1) according to anyone of the preceding claims, in particular a cassette (1) fixedly disposed of an injector tube (22).

12. Injector device (21) according to claim 11,
**characterized in that**
the injector device (21) has an injector tip (24) formed with a perforation edge (27), with which the foil (10, 41) of the cassette (1) facing the injector tip (24) is automatically severable upon arrangement of the injector tip (24) on an injector tube (22) of the injector device (21).

13. Injector device (21) according to claim 11 or 12,
**characterized in that**
injector device (21) has a piston (23) for pushing the intraocular lens (3) out of the cassette (1), which has a perforation means, by which upon the piston (23) striking a foil (16) of the cassette (1) facing the piston (23), this foil (16) is automatically severable.

14. Injector device (21) according to any one of claims 11 to 13,
**characterized in that**
the cassette (1) is steam sterilized and the injector tip (24) and the injector tube (22) are sterilized with ethylene oxide.

15. Injector device (21) according to any one of claims 11 to 14,
**characterized in that**
it is disposed in a closed transport container together with the cassette (1) disposed thereon.

## Patentansprüche

1. Kassette (1) für eine Intraokularlinse (3), die einen Aufnahmebehälter (2) für die Intraokularlinse (3) aufweist, der eine Vorderseite (6) und eine Rückseite (7) in der axialen Richtung (A) des Aufnahmebehälters (2) hat, wobei
die Vorderseite (6) eine Öffnung (8) aufweist, die durch eine an dem Aufnahmebehälter (2) befestigte Folie (10) als Abdeckung geschlossen wird, und/oder die Rückseite (7) eine Öffnung (9) aufweist, die durch eine an dem Aufnahmebehälter (2) befestigte Folie (16) als Abdeckung geschlossen wird,
**dadurch gekennzeichnet, dass** die Folie (10, 16) mit einem Abreißelement (14) verbunden ist, das relativ zu dem Aufnahmebehälter (2) beweglich ist, um die Folie (10, 16) von dem Aufnahmebehälter (2) abzureißen, und
die Folie (10, 16) integral gebildet ist und eine Biegung (13, 18) an dem Ende aufweist, das von dem Abreißelement (14) weg weist, und wobei eine zweite Folienschicht (12, 19) bezogen auf die Öffnung (8, 9) des Aufnahmebehälters (2) außerhalb an dem freien Ende, das von der Biegung (13, 18) weg weist, mit dem Abreißelement (14) verbunden ist.

2. Kassette (1) nach Anspruch 1, **dadurch gekennzeichnet, dass** die Folie eine coextrudierte Folie (10, 16) ist.

3. Kassette (1) nach Anspruch 2, **dadurch gekennzeichnet, dass** die coextrudierte Folie (10, 16) Aluminium umfasst.

4. Kassette (1) nach Anspruch 2 oder 3, **dadurch gekennzeichnet, dass** die coextrudierte Folie (10, 16) Polypropylen umfasst.

5. Kassette (1) nach einem der vorhergehenden Ansprüche, **dadurch gekennzeichnet, dass** die Folie (10, 16) eine Dicke zwischen 0,3 mm und 6 mm, insbesondere 0,3 mm und 1 mm, insbesondere 0,5 mm aufweist.

6. Kassette (1) nach einem der vorhergehenden Ansprüche, **dadurch gekennzeichnet, dass** die Folie (10, 16) in zwei Schichten auf einer Öffnung (8, 9) gebildet ist.

7. Kassette (1) nach einem der vorhergehenden Ansprüche, **dadurch gekennzeichnet, dass** die Folie (10, 16) an den Aufnahmebehälter (2) wärmegeschweißt ist.

8. Kassette (1) nach einem der vorhergehenden Ansprüche, **dadurch gekennzeichnet, dass** ein verschiebbares elastisches Dämpferelement (28) in dem Aufnahmebehälter (2) auf der Öffnung (9) angeordnet ist, die eine Eingangsseitenöffnung für einen Kolben (23) an einer Injektorvorrichtung (21) bildet.

9. Kassette (1) nach einem der vorhergehenden Ansprüche, **dadurch gekennzeichnet, dass** die Folie (10, 16) eine definierte Perforationsstruktur (26, 29) aufweist.

10. Kassette (1) nach Anspruch 9, **dadurch gekennzeichnet, dass** die Perforationsstruktur (26, 29) mindestens zwei gekreuzte Linie einschließt.

11. Injektorvorrichtung (21) für eine Intraokularlinse (3), die eine Kassette (1) gemäß einem der vorhergehenden Ansprüche einschließt, insbesondere eine Kassette (1), die fest an einem Injektorrohr (22) angeordnet ist.

12. Injektorvorrichtung (21) nach Anspruch 11, **dadurch gekennzeichnet, dass** die Injektorvorrichtung (21) eine Injektorspitze (24) aufweist, die mit einem Perforationsrand (27) gebildet ist, mit dem die Folie (10, 41) der Kassette (1), die zu der Injektorspitze (24) weist, automatisch bei Anordnung der Injektorspitze (24) auf einem Injektorrohr (22) der Injektorvorrichtung (21) trennbar ist.

13. Injektorvorrichtung (21) nach Anspruch 11 oder 12, **dadurch gekennzeichnet, dass** die Injektorvorrichtung (21) einen Kolben (23) aufweist, um die Intraokularlinse (3) aus der Kassette (1) zu schieben, der ein Perforationsmittel aufweist, mit dem, wenn der Kolben (23) auf eine Folie (16) der Kassette (1) trifft, die zu dem Kolben (23) weist, diese Folie (16) automatisch trennbar ist.

14. Injektorvorrichtung (21) nach einem der Ansprüche 11 bis 13, **dadurch gekennzeichnet, dass** die Kassette (1) dampfsterilisiert ist, und die Injektorspitze (24) und das Injektorrohr (22) mit Ethylenoxid sterilisiert sind.

15. Injektorvorrichtung (21) nach einem der Ansprüche 11 bis 14, **dadurch gekennzeichnet, dass** sie in einem geschlossenen Transportbehälter zusammen mit der darauf angeordneten Kassette (1) angeordnet ist.

## Revendications

1. Cassette (1) destinée à une lentille intraoculaire (3), qui comporte un contenant de réception (2) pour la lentille intraoculaire (3), qui a une face avant (6) et arrière (7) dans la direction axiale (A) du contenant de réception (2), dans laquelle la face avant (6) possède une ouverture (8), qui est fermée par une feuille (10) fixée au contenant de réception (2) sous la forme d'un couvercle, et/ou la face arrière (7) possède une ouverture (9), qui est fermée par une feuille (16) fixée au contenant de réception (2) sous la forme d'un couvercle,
**caractérisée en ce que**
ladite feuille (10, 16) est reliée à un élément détachable (14), qui est mobile par rapport au contenant de réception (2) pour détacher la feuille (10, 16) du contenant de réception (2), et
la feuille (10, 16) est formée d'un seul bloc et possède une courbure (13, 18) au niveau de l'extrémité partant de l'élément détachable (14), et une seconde couche de feuille (12, 19) à l'extérieur par rapport à l'ouverture (8, 9) du contenant de réception (2) est reliée à l'élément détachable (14) au niveau de l'extrémité libre partant de la courbure (13, 18).

2. Cassette (1) selon la revendication 1,
**caractérisée en ce que**
ladite feuille est une feuille coextrudée (10, 16).

3. Cassette (1) selon la revendication 2,
**caractérisée en ce que**
ladite feuille coextrudée (10, 16) comprend de l'aluminium.

4. Cassette (1) selon la revendication 2 ou 3,
**caractérisée en ce que**
ladite feuille coextrudée (10, 16) comprend du polypropylène.

5. Cassette (1) selon l'une quelconque des revendications précédentes,
**caractérisée en ce que**
la feuille (10, 16) a une épaisseur comprise entre 0,3 mm et 6 mm, en particulier 0,3 mm et 1 mm, en particulier 0,5 mm.

6. Cassette (1) selon l'une quelconque des revendications précédentes,
**caractérisée en ce que**
ladite feuille (10, 16) est formée en deux couches sur une ouverture (8, 9).

7. Cassette (1) selon l'une quelconque des revendications précédentes,
**caractérisée en ce que**
ladite feuille (10, 16) est soudée à chaud au contenant de réception (2).

8. Cassette (1) selon l'une quelconque des revendications précédentes,
**caractérisée en ce que**
un élément amortisseur élastique déplaçable (28) est disposé dans le contenant de réception (2) sur l'ouverture (9) constituant une ouverture du côté entrée pour un piston (23) sur un dispositif d'injecteur (21).

9. Cassette (1) selon l'une quelconque des revendications précédentes,
**caractérisée en ce que**
ladite feuille (10, 16) a une structure perforée (26, 29) définie.

10. Cassette (1) selon la revendication 9,
**caractérisée en ce que**
la structure perforée (26, 29) comprend au moins deux lignes se croisant.

11. Dispositif d'injecteur (21) destiné à une lentille intraoculaire (3), comprenant une cassette (1) selon l'une quelconque des revendications précédentes, en particulier une cassette (1) disposée sur un tube d'injecteur (22).

12. Dispositif d'injecteur (21) selon la revendication 11,
**caractérisé en ce que**
le dispositif d'injecteur (21) possède un embout (24) d'injecteur pourvu d'un bord de perforation (27), avec lequel la feuille (10, 41) de la cassette (1) tournée vers l'embout (24) d'injecteur est automatiquement séparable lors de l'agencement de l'embout (24) d'injecteur sur un tube (22) d'injecteur du dispositif d'injecteur (21) .

13. Dispositif d'injecteur (21) selon la revendication 11 ou 12,
**caractérisé en ce que**
le dispositif d'injecteur (21) possède un piston (23) permettant de pousser la lentille intraoculaire (3) hors de la cassette (1), qui possède un moyen de perforation, par lequel lorsque le piston (23) vient frapper une feuille (16) de la cassette (1) tournée vers le piston (23), cette feuille (16) est automatiquement séparable.

14. Dispositif d'injecteur (21) selon l'une quelconque des revendications 11 à 13,
**caractérisé en ce que**
la cassette (1) est stérilisée à la vapeur et l'embout (24) d'injecteur et le tube (22) d'injecteur sont stérilisés à l'oxyde d'éthylène.

15. Dispositif d'injecteur (21) selon l'une quelconque des revendications 11 à 14,
**caractérisé en ce que**
il est disposé dans un contenant de transport fermé conjointement avec la cassette (1) disposée sur celui-ci.
